Europäisches Patentamt

European Patent Office

Office européen des brevets

(19)

(11) **EP 1 077 216 B1**

(12) **EUROPEAN PATENT SPECIFICATION**

(45) Date of publication and mention
of the grant of the patent:
**19.10.2005 Bulletin 2005/42**

(51) Int Cl.$^{7}$: **C07K 5/06**

(21) Application number: **00307042.2**

(22) Date of filing: **17.08.2000**

(54) **Crystallization of alpha-L-aspartyl-L-phenylalanine methyl ester from supersaturated solution**

Kristallisierung von Alpha-L-Aspartyl-L-Phenylalaninmethylester aus übersättigten Lösungen

Crystallisation de l'ester méthylique de l'alpha-L-aspartyl-L-phenylalanin à partir de solutions sursaturées

(84) Designated Contracting States:
**AT BE CH CY DE DK ES FI FR GB GR IE IT LI LU MC NL PT SE**

(30) Priority: **18.08.1999 US 149570 P**

(43) Date of publication of application:
**21.02.2001 Bulletin 2001/08**

(73) Proprietor: **NutraSweet IP Holdings, Inc.**
**Chicago Illinois 60654 (US)**

(72) Inventor: **Choi, Cheong-Song**
**Songpa-ku, Seoul 138-240 (KR)**

(74) Representative: **W.P. Thompson & Co.**
**Coopers Building**
**Church Street**
**Liverpool L1 3AB (GB)**

(56) References cited:
**EP-A- 0 484 769**

• **S KISHIMOTO & M NARUSE: "A process development for the bundling crystallization of aspartame" JOURNAL OF CHEMICAL TECHNOLOGY AND BIOTECHNOLOGY. (INTERNATIONAL JOURNAL OF BIOTECHNICAL AND CHEMICAL PROCESSES), vol. 43, no. 1, 1988, pages 71-82, XP002153917 ELSEVIER APPLIED SCIENCE PUBLISHERS. BARKING., GB ISSN: 0268-2575**
• **T MORI ET AL.: "Spherulitic crystallization of aspartame from aqueous solution in a two-dimensional cell" JOURNAL OF CRYSTAL GROWTH., vol. 133, 1993, pages 80-86, XP002153918 NORTH-HOLLAND PUBLISHING CO. AMSTERDAM., NL ISSN: 0022-0248**

**Description**

Field of the Invention

The present invention relates to a method for producing α-L-aspartyl-L-phenylalanine methyl ester ("α-APM" or "aspartame"). More particularly, this invention pertains to the crystallization of α-APM from a metastable supersaturated solution.

Description of the Related Art

Aspartame or α-APM is a well-known low calorie sweetener that is widely used in a variety of food and beverage products. Various methods for crystallizing α-APM have been proposed, including cooling crystallization.

A typical cooling crystallization process begins with a hot unsaturated feed solution being charged to a reactor equipped with a heat exchanger for cooling. A supersaturated state is created shortly after cooling begins. Nucleation then occurs, crystals grow, and supersaturation is depleted. Because the crystal size distribution is dependent on the supersaturation profile of the crystallization process as it occurs, the cooling rate is of critical importance in determining the resulting crystal size distribution. Therefore, the cooling rate must be closely monitored and controlled in order to achieve optimal crystal size distribution.

However, such control can be difficult to achieve when the crystallization method utilizes an agitated crystallizer equipped with a cooling jacket or coil. In such a system, crystals are deposited on the cooling surface of the crystallizer. This has the effect of drastically reducing the heat transfer coefficient between the surface and solution, thereby reducing the efficiency of the cooling process and making it more difficult to control the cooling rate, and thus the crystal size distribution. Moreover, the crystals produced by such a method often display poor filterability, which makes further processing of the precipitated crystals difficult.

Another process known in the art for preparing α-APM crystals comprises cooling an aqueous solution of α-APM through conduction heat transfer without mechanical agitation. Such a method produces a sherbet-like pseudo-solid phase of α-APM crystals. The crystals produced by such a method demonstrate better filterability than those produced by the conventional cooling process described above wherein the mixture is stirred during crystallization. However, a crystallization method involving cooling through heat transfer without mechanical agitation has the disadvantage of requiring a very long operation time.

Kishimoto and Naruse describe, in *J. Chem. Tech. Biotechnol. 1988, 43, 71-82,* a process development for the bundling crystallization of aspartame, describing the development of a new type of crystallizer for large-scale crystallization of aspartame.

Mori *et al* describe, in *Journal of Crystal Growth 133 (1993) 80-86,* be spherulitic crystallization of aspartame from aqueous solution in a two-dimensional cell.

EP-A-0976762 discloses a method of crystallising aspartame by heat exchanging an aspartame solution with a coolant passed in the opposite direction.

DE-A-19912699 discloses a method for controlling crystal size during continuous mass crystallization by controlling the parameters of the crystal seed product.

Summary of the Invention

Accordingly, in a first embodiment of the present invention, a method of producing crystals of α-L-aspartyl-L-phenylalanine methyl ester ("α-APM") is provided, the method including the steps of forming a supersaturated solution of α-APM, initiating nucleation of α-APM crystal nuclei with use of a peristaltic pump, thereby forming a suspension of crystal nuclei in the supersaturated solution, and growing the α-APM crystal nuclei in the supersaturated solution, without substantial formation of new crystal nuclei.

In aspects of this embodiment, the step of forming the supersaturated solution includes preparing a non-saturated α-APM solution and cooling the non-saturated solution to form the supersaturated solution. The concentration of the non-saturated α-APM solution may preferably be in the range from about 1.5 wt. % to about 7.0 wt. %, and more preferably from about 3.0 wt. % to about 5.5 wt. %. The temperature of the non-supersaturated α-APM solution may preferably be in the range from about 35 °C to about 85 °C before cooling, more preferably from about 55 °C to about 75 °C before cooling. The cooling step may be conducted at less than a maximum allowable undercooling at a concentration, or by heat exchanging with a coolant. The solution may be an aqueous solution, and the concentration of α-APM in the supersaturated aqueous solution prior to initiating nucleation may be greater than about 3.5wt. %. The step of initiating nucleation is conducted by mechanical forcing of the solution by a peristaltic pump.

The step of initiating nucleation may be conducted at a solution temperature ranging from about 28°C to about 80°C, more preferably about 48° to about 66°C.

In further aspects of this embodiment, the step of growing the α-APM crystal nuclei may include a spontaneous growing stage after the initiation of nucleation and a forced growing stage after the spontaneous growing stage. The spontaneous growing stage may proceed at a supersaturated solution temperature ranging from about 45 ° C to 60°C. The forced growing stage may include cooling the suspension of crystal nuclei in the supersaturated solution. The cooling may be conducted at a temperature ranging from about 5 °C to 60 °C, in a batch or continuous mode operation, or for a time of from about 50 to about 500 min. The suspension of crystal nuclei in the supersaturated solution may be stirred during the cooling, such as by an anchor type impeller or a ribbon type impeller, and a scraper may be attached to the impeller. The stirring may be conducted at from about 2 rpm to about 100 rpm. The suspension of crystal nuclei in the supersaturated solution could not be in contact with a gaseous atmosphere before the cooling.

In yet other aspects of this embodiment, the method further includes conducting a solid-liquid separation after growing the $\alpha$-APM crystal nuclei in the supersaturated solution to form $\alpha$-APM crystals. The $\alpha$-AMP crystals may then be dried under elevated temperature.

In a second embodiment of the present invention, a method of continuously crystallizing $\alpha$-APM dissolved in a solution is provided, the method including continuously feeding a supersaturated solution of $\alpha$-APM to a peristaltic pump, continuously generating crystal nuclei of $\alpha$-APM in the supersaturated solution by rolling action of the pump to from a suspension of crystal nuclei in the supersaturated solution, continuously transferring the suspension to a crystallizer, and continuously growing the crystal nuclei by cooling the suspension in the crystallizer without substantial formation of new nuclei.

In aspects of this embodiment, the step of continuously feeding may include continuously cooling a non-saturated solution to form a supersaturated solution. The step of continuously growing may include continuously stirring the suspension or continuously scraping an internal surface of the crystallizer. The stirring may be provided by an anchor type impeller or a ribbon type impeller. The method may further include continuously withdrawing the suspension from the crystallizer.

In a third embodiment of the present invention, an apparatus for use in crystallization of $\alpha$-APM dissolved in a solvent is provided, the apparatus including a source of a supersaturated solution of $\alpha$-APM, a peristaltic pump in fluid communication with the supersaturated solution, and a crystallizer in fluid communication with the pump.

In aspects of this embodiment, the apparatus may further include a transfer line, optionally equipped with a heat exchanger, in fluid communication with the source and the pump. The crystallizer, optionally equipped with a heat exchanger, may include a cooling container adapted to receive and cool a suspension of crystal nuclei from the pump The apparatus may further include a stirrer, such as an anchor type impeller or a ribbon type impeller, or a scraper. The apparatus may further include a transfer line in fluid communication with the crystallizer and the pump.

Other aspects and features of the present invention will be further discussed in detail with reference to the following drawings and description.

Brief Description of the Drawings

Figure 1 illustrates a crystallization system of a preferred embodiment of the present invention.

Figure 2 illustrates a solubility-supersolubility diagram demonstrating the relationship between temperature and concentration of a solute in solution during cooling, in the absence of a solid phase.

Figure 3 illustrates a solubility-supersolubility diagram demonstrating the relationship between temperature and concentration of a solute in solution during the controlled crystallization process of the present invention.

Figure 4 illustrates conceptual operation of the nucleation pump of the preferred embodiment shown in Figure 1.

Detailed Description of the Preferred Embodiment

Under the current state of the art in $\alpha$-APM crystallization processes, one must choose between either a method which does not require a long crystallization time, but which produces crystals of inferior physical properties, or a method which produces crystals of superior physical properties, but which requires a long crystallization time. A short crystallization time and crystals of superior physical properties are both desired attributes for a crystallization process, however. Therefore, a method for crystallizing $\alpha$-APM of good physical properties which does not require an unduly long crystallization time is desirable. The use of the novel method of the present invention achieves this by permitting the operation of an $\alpha$-APM crystallization process which does not require an unduly long crystallization time but which also avoids the uncontrolled spontaneous crystallization which leads to inferior physical properties of the crystals produced.

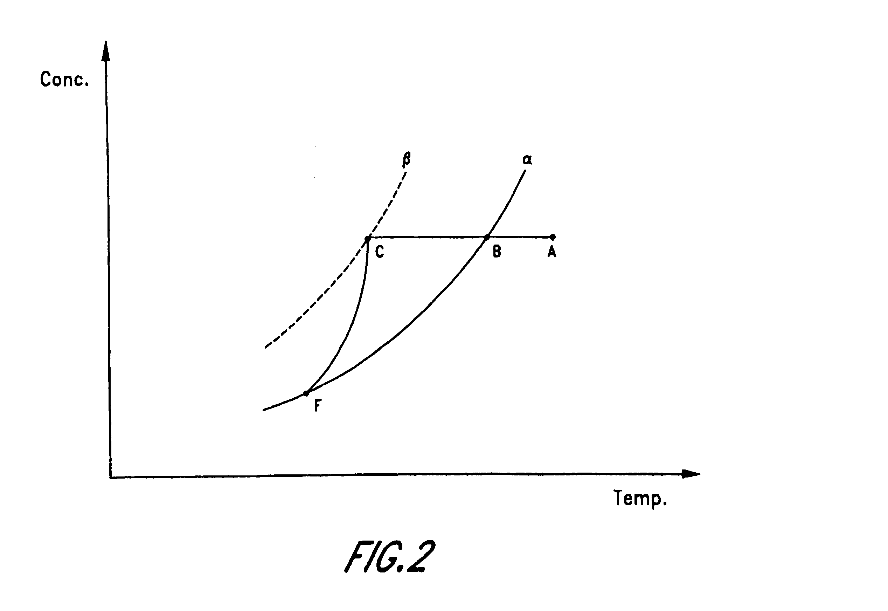

The relationship between temperature and concentration of a solute in solution with regard to crystal formation is represented in Figure 2. Curve $\alpha$ represents the solubility of $\alpha$-APM. Curve $\beta$, which runs approximately parallel with the solubility curve $\alpha$, is the boundary of the metastable region. Sometimes this curve is referred to as the curve of maximum permissible supersaturation or the limit of unstable supersaturation. The area between curves $\alpha$ and $\beta$ is defined as a metastable supersaturated region, in which spontaneous crystallization is improbable, but can occur if nucleation is initiated, or if seed crystals are introduced. The nucleation can be initiated by applying a form of energy to a solution in a metastable supersaturated solution.

In a conventional agitated crystallization process, mechanical stirring initiates the nucleation from a supersaturated $\alpha$-APM solution in the metastable supersaturated region. The nucleation caused by such mechanical stirring or agitation is substantial and extremely drastic, resulting in fine α-APM crystals characterized as needlelike crystals. A likely explanation is that when an α-APM metastable supersaturated solution is agitated, too many α-APM nuclei are generated instantly and consume most of the solute that is over-dissolved with reference to the solubility level at a given temperature. As a result, the solution does not have much solute that can contribute to the growth of the small-sized nuclei at the temperature. The solution can be cooled to further precipitate the solute onto the existing crystals as seeds. However, since there are substantially too many fine crystals suspended in the solution, precipitation of the solute caused by the cooling cannot significantly grow the crystals and increase their sizes. Also, even if the mechanical stirring is slow or light, the generation of nuclei still occurs in a substantial and uncontrollable manner.

In order to avoid such drastic and substantial nucleation and to produce coarse crystals, a non-stirring crystallization of α-APM was suggested. The non-stirring crystallization is conceptually described now in reference to Figure 2. Consider, for example, a solution wherein the concentration and temperature are specified by point A. On gradually lowering the temperature, one arrives at point B on the solubility curve α. If the system is free from solid phase, further lowering of the temperature does not lead to crystallization until point C is reached. At point C, the first crystals begin to appear, i.e., nucleate and grow, until the concentration diminishes to the equilibrium point of solubility given by point F. Further gradual cooling leads to movement along the equilibrium curve α. Quicker cooling would correspond to displacement paths between the curves α and β. Rapid cooling, i.e., movement along curve β, would continuously produce new crystal nuclei. However, this non-stirring crystallization takes a very long time to produce coarse α-APM crystals, as mentioned above.

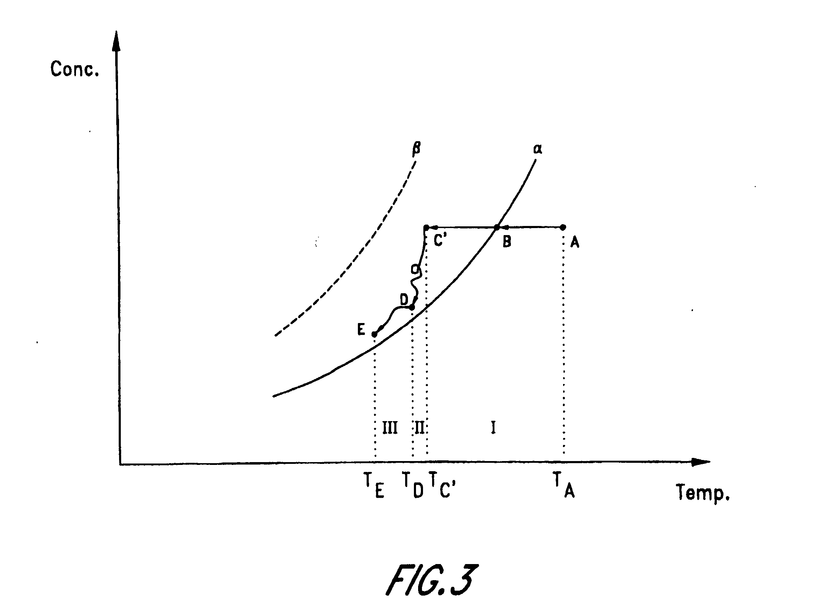

The present invention provides a new concept for crystallisation of a solute dissolved in a solution. Applying this concept to the crystallization of α-APM, although not limited thereto, coarse α-APM crystals are produced in a significantly shorter time on an industrial scale. Figure 3 represents the principle of the controlled crystallization in accordance with the present invention. A solution at point A is prepared and cooled to a point C', representing a supersaturated solution in the metastable region. When the solution reaches point C', a certain form of mechanical energy is inputted to the solution, which initiates the nucleation in a controlled manner. The nucleation of α-APM is controlled such that the rate of generation of nucleation is significantly reduced in comparison to that in the stirring crystallization. Only a part of the over-dissolved α-APM at a given temperature is consumed and a constant number of α-APM nuclei is generated in the nucleation, by which the solution moves from point C' to point D in Figure 3. Then, α-APM crystals spontaneously grow from these existing nuclei without significant additional nucleation, resulting in the change from point D to point E.

At a given temperature or concentration, a metastable solution becomes more stable as it gets closer to the solubility curve α, and vice versa. In other words, a metastable solution close to the curve α is less likely to nucleate than another metastable solution close to the curve β when the other conditions are the same. The controlled nucleation and following spontaneous crystal growth in the metastable region move the solution toward the solubility curve α, the solution gets stabilized. At the point E, the concentration of the solute is close to the solubility level and the solution is very stable.

The solution at point E still contains a considerable amount of solute. In order to produce coarser crystals and improve the yield of the crystallization, further crystallization proceeds. The α-APM slurry, the mixture of the solution and the crystals suspended therein, is cooled. As the temperature decreases, the solution at point E moves to the left toward the β within the metastable region. At a given temperature, some of the over-dissolved solute with reference to the solubility curve α can be precipitated. As the slurry becomes cooler, the existing crystals in the slurry continuously grow by consuming the over-dissolved α-APM solute. The cooling and continuous consumption of the solute lead to movement along the solubility curve α. New nucleation does not occur in an appreciable manner during the cooling since the solution is already in a stable state. As such, mechanical energy input such as agitation or stirring can be applied to the slurry without significant generation of additional nuclei.

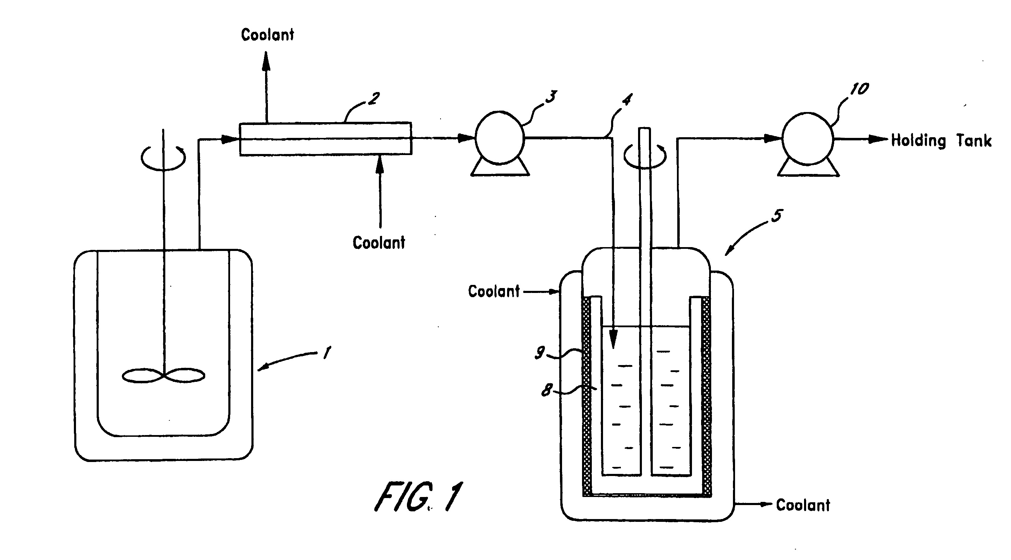

Now referring to Figures 1 and 3, a preferred embodiment of the controlled crystallization in accordance with the present invention is described. A non-saturated aqueous α-APM solution is prepared by dissolving α-APM in water in a dissolver 1 as shown in Figure 1 or as a result of the synthesis of α-APM. The solution is transferred to and cooled in a heat exchanger 2. The cooled metastable solution is fed to a nucleation pump 3, in which crystallization takes place initially through the formation of new nuclei. The nuclei then grow by consuming the solute in the metastable solution in a transfer line 4. The α-APM slurry is transferred to a crystallizer 5, in which further crystallization continues with coolant circulation and agitation.

The concentration and temperature of the solution is adjusted, for example in the dissolver 1. The concentration of the solution ranges from about 1.5 wt. % to about 7.0 wt. %, and the temperature in the concentration range is from about 35 °C to about 85 °C. Preferably, the concentration of the solution is from about 3.0 wt. % to about 5.5 wt. % at a temperature ranged from about 55 °C to about 75 °C, and more preferably the concentration is from about 3.5 wt. % to about 5.0 wt. % at from about 60 °C to about 70 °C. The aqueous solution is represented by point A in Figure 3.

As an example, an aqueous solution of about 4.0 wt. %

$\alpha$-APM at a temperature of about 65 °C is prepared. This solution is fed to the heat exchanger 2 positioned between the dissolver 1 and the nucleation pump 3 as shown in Figure 1. Cooling proceeds with no change in $\alpha$-APM concentration until a certain level of supersaturation (given by point C' in Figure 3) in the metastable region is reached. During this cooling process (represented as Stage I in Figure 3), no spontaneous nucleation takes place. This cooling is controlled such that the solution move too far left to hit the curve $\beta$ of maximum permissible supersaturation, which will initiate the nucleation.

The relationship between the maximum allowable undercooling, $\Delta T_{max}$ [°C], and the cooling rate, $-dT/dt$ [°C/hr], for unseeded aqueous solutions of $\alpha$-APM can be expressed as:

$$-dT/dt = 0.231 * (\Delta T_{max})^{2.266}$$

The maximum allowable undercooling is the temperature difference between the curves $\alpha$ and $\beta$ at a given concentration. This result can be used for the practical purpose of assessing the width of the metastable region for $\alpha$-APM crystallization. In this embodiment of the present invention the heat exchanger 2 is designed such that the cooling is conducted less than the maximum allowable undercooling. The solution is cooled to a temperature in the range of from about 28 °C to about 80 °C with concentration from about 1.5 wt. % to about 7.0 wt. %. Preferably, the temperature after the heat exchange is from about 48 °C to about 66 °C at a concentration ranged from about 3.0 wt. % to about 5.5 wt. %., more preferably from about 51 °C to about 62 °C at a concentration from about 3.5 wt. % to about 5.0 wt. %. The coolant heat-exchanging with the solution has a temperature ranged from about 0 °C to about 40 °C. The coolant temperature is preferably from about 5 °C to about 30 °C, more preferably from 10 °C to about 25 °C.

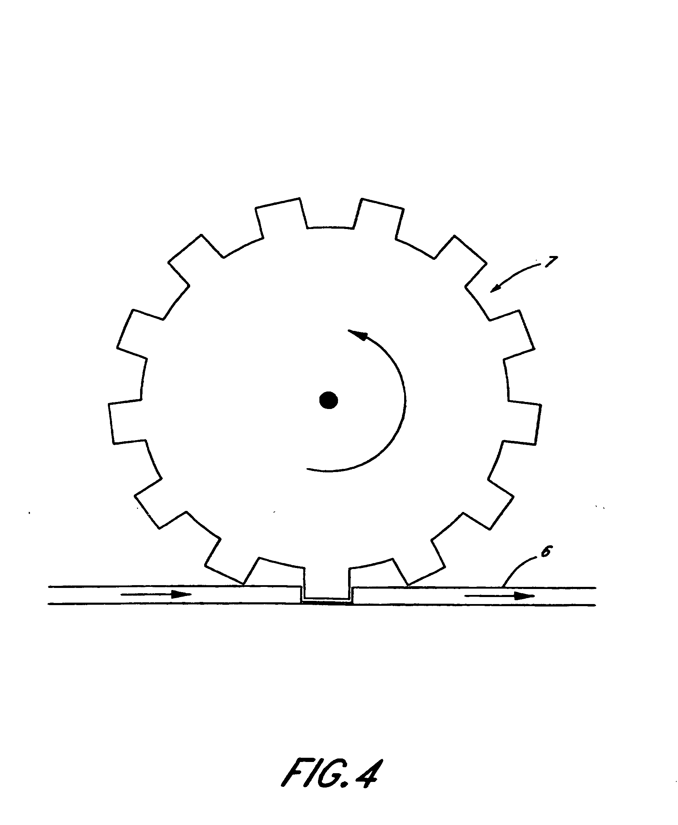

The instant that the solution, at temperature $T_{C'}$ in a metastable state, enters the nucleation pump 3, the nucleation stage (designated as Stage II in Figure 3) begins due to mechanical forcing from the rolling action of the nucleation pump 3. The pump 3 includes a peristaltic pump, commonly referred to as a squeeze pump. Figure 4 conceptually illustrastes the peristaltic pump. In the pump, peristaltic flow is created in a tube 6 by a rotating roller mechanism 7 that alternately compresses and relaxes a section of the tubing 6. As the compressed section recovers its shape, suction is created drawing in fluid which is then pushed forward by the next advancing roller. The pump action initiates nucleation and crystallization, thereby decreasing the concentration of $\alpha$-APM in solution within the region of metastability. In this stage, concentration of the $\alpha$-APM solution decreases rapidly (to point D of Figure 3) as result of consumption of the solute by both nucleation and crystal growth of the formed nuclei in the pump.

When the solution containing crystals formed in the squeezed tubing part leaves the pump 3, substantially no further nucleation occurs and growth of the nuclei occurs in the upstream transfer line 4 of the crystaller 5. The temperature in Stage II of Figure 3 increases slightly due to the release of latent heat of crystallization, but cooling by heat exchange with the surrounding environment produces an overall reduction in the temperature of the mixture of crystals in solution. By the time the mixture of crystals in solution reaches the crystallizer 5, the concentration of solute is lowered close to the solubility level (point E in Figure 3). Nucleation of $\alpha$-APM ceases and a constant number of stable $\alpha$-APM crystals are introduced into the crystallizer 5. The temperature of the mixture of crystals in solution upon introduction into the crystallizer 5 is from about 45 °C to about 60 °C, although not limited thereto. Preferably, the temperature is in the range of from about 48°C to about 55°C, more preferably 50°C to 52°C, but higher temperatures may also be used.

While the slurry is entering into the crystallizer 5, the slurry's exposure to environmental air or atmosphere is preferably avoided. If the slurry contacts the air, it may trap and carry some of the gaseous phase into the slurry contained in the crystallizer 5, causing additional nucleation in the crystallizer 5 which generates fine nuclei. Substantial new formation of fine nuclei deteriorates the production of coarse $\alpha$-APM crystals. Thus, in this preferred embodiment, the transfer line 4 is extended to the bottom of the crystallizer 5 at least at the time of charging the slurry into an empty crystallizer. Alternatively, the transfer line 4 can simply be extended below the top surface of the slurry if some of the slurry is contained in the crystallizer 5.

After the solution has undergone the controlled crystallization as described above, the mixture of $\alpha$-APM crystals in solution is subjected to further crystallization by cooling the slurry and allowing the crystals in the feed solution to grow. Cooling in the crystallizer 5 takes place through the indirect heat exchange with a coolant circulating in an external cooling jacket, as shown in Figure 1. The temperature of the coolant entering the cooling jacket is from about 0 °C to about 25 °C. Preferably, it ranges from about 2 °C to about 20 °C, more preferably from about 5 °C to about 15 °C. Such cooling can be carried out with or without stirring of the slurry. Also the cooling crystallization can be carried out in either batch mode or continuous mode operation.

In the preferred embodiment of the present invention, the stirring of the slurry is used during the cooling without further substantial generation of nuclei. Any type of stirrers or mixers can be used to provide the stirring to the slurry in the crystallizer 5. Stirrers, however, may break up some of the crystals suspended in the solution by impact. Preferably, the stirrers, which either do not break the crystals or cause insignificant breakage of the crystals, are used. Exemplary stirrers include an anchor type impeller and, in the case of the industrial scale process, a ribbon type impeller. The crystallizer 5 shown in Figure 1 has an anchor type impeller 8. A scraper 9 is preferably attached to the edges of the anchor or ribbon impeller 8 and keeps the interior walls of the crystallizer 5 free of adherent α-APM crystals by continuously scraping the crystallized deposit from the cold surface of the crystallizer wall. Alternatively, a separate scraper and stirrer may be used in the present invention. The rotational speed of the stirrer 8 can be determined depending on the size of the crystallizer and other parameters to effect significant mixing of the slurry contained in the crystallizer 5. However, the stirrer rotates at a speed of from about 2 rpm to about 100 rpm, preferably, the speed is from about 3 rpm to about 70 rpm, more preferably from about 5 rpm to about 50 rpm.

The stirring homogenizes the slurry of the α-APM crystals in the solution and enhances the heat transfer to the entire slurry. This significantly reduces the time required for this further precipitation by cooling of the α-APM solute dissolved in the solution. In conducting the crystallizing step, it is advantageous to minimize the amount of crystals adhering to the crystallizer wall. By minimizing crystal adherence to the walls, the heat transfer layer may be more extensively renewed. In sum, combined with a scraper renewing the heat transferring surface, the stirring of the slurry significantly enhances the heat transfer efficiency and allows the continuous operation of this further crystallization for an expanded period of time without stopping for removing scales.

If the process is to be conducted in a continuous mode, then in the initial stages of continuous mode operation when the mixture of crystals is first fed to the crystallizer 5, an optional aging step may be conducted. In the aging step, the initial charge of mixture of crystals in solution is allowed to "age" for a certain period of time without introduction of any additional solution into the crystallizer 5. After this initial aging period, the mixture of crystals in solution is introduced into the crystallizer 5 at the same rate that solution containing grown α-APM crystals is withdrawn from the crystallizer 5 and transferred to the receiving tank (not shown) by the action of a discharge pump 10. The mean residence time of mixture in the crystallizer 5 is in the range of from about 50 to 500 minutes, preferably 60 to 180 minutes, when operating in continuous mode. The temperature of the mixture during crystallization when operating in a continuous mode is in the range of from about 5°C to about 45°C, preferably from 6 °C to about 35 °C, more preferably from about 6 °C to 20°C.

The process may also be operated in batch mode. In batch mode, an amount of solution is introduced into the crystallizer 5 and allowed to cool without the addition or withdrawal of solution. After the crystallization step is completed, the solution is then withdrawn and transferred to the receiving tank (not shown). When operating in batch mode the temperature and residence time in the crystallizer 5 need not be as tightly controlled as when operating in continuous mode, thereby allowing greater flexibility in processing conditions.

After the crystallization discussed above, the α-APM slurry is subject to a solid-liquid separation. For instance, gravity filtration, pressure filtration, centrifuge filtration, reduced pressure filtration or evaporation of solvent can be used to separate the α-APM from its mother liquor. The solid-liquid separation is followed by a drying stage, which removes moisture retained in the separated solid α-APM. Normally, the solid α-APM is dried under elevated temperature.

By utilizing the crystallization method of the present invention, it is possible to obtain α-APM crystals with properties superior to those of crystals produced by conventional methods. The crystals demonstrate good filterability and solid-liquid separation. The crystals produced by the crystallization in accordance with the present invention are discrete and coarse crystals, needlelike in shape but thicker than those produced by the prior art cooling crystallization method wherein the solution is mixed. Also, the crystallization system apparatus is more compact than in conventional processes because of the remarkable reduction in the required residence time of the slurry. Thus, the present invention provides a method for α-APM crystallization that is markedly advantageous from economic viewpoint. The present invention is effective in batch mode as well as in continuous operation mode.

The following Examples illustrate the present invention in further detail. Those of skill in the art will recognize that there are numerous variations and modifications of this invention that are encompassed by its scope. Accordingly, the following Examples should only be considered illustrative of the present invention and not deemed to limit its scope. Some of the parentheses below are to correspond the devices and apparatuses used in the Examples to those shown in Figure 1.

EXAMPLE I

An aqueous solution of α-APM entered the upstream transfer line (the transfer line between the dissolver 1 and the heat exchanger 2) to the crystallizer (the crystallizer 5). Tap water at a temperature ranging from about 7 °C to 8 °C was circulated through a cooling jacket (the heat exchanger 2) surrounding the transfer line. An aqueous solution of about 4 wt. % α-APM having a temperature of 65 °C was pulled through the transfer line into a pump (nucleation pump 3) at a rate of 20 ml/min. Crystals of α-APM began to form in the solution as it was forced through the nucleation, forming a slurry. The slurry (at a temperature of about 50 °C to 52 °C) was continuously fed to the crystallizer. The crystallizer had a volume of 3 liters and was equipped with a cooling jacket and anchor (anchor type impeller 8) with scraper (scraper 8). The impeller on the anchor was set to a rotation of 50 rpm. A coolant having a temperature of 5 °C was circulated through the cooling jacket of the crystallizer.

Once the crystallizer was entirely filled with slurry, charging of slurry to the crystallizer was stopped and the slurry was aged for 30 min to stabilize the crystallization system. After aging, charging of slurry to the crystallizer was reinitiated at a rate of 50 ml/min, and slurry was withdrawn from the crystallizer at the same rate, 50 ml/min, so as to keep a constant volume of slurry in the crystallizer. The average residence time of slurry in the crystallizer was about 60 minutes, and the temperature of the slurry during crystallization under steady state conditions was in the range of 6 °C to 12 °C. The crystallization process was carried out for two consecutive days, during which time scale was never generated on the sides of the crystallizer. The slurry thus produced demonstrated excellent filterability, i.e., the water content of the resulting wet crystals of $\alpha$-APM ranged from 37 to 45 wt.%.

EXAMPLE II

An industrial scale crystallization system as illustrated in Figure 1 was set up. The dissolver 1 corresponds to a tank for containing an aqueous $\alpha$-APM solution, which was neutralized from $\alpha$-APM·HCl in a final stage of the $\alpha$-APM synthesis. The concentration of the $\alpha$-APM solution was adjusted to 4.8 - 5.0 wt.% at the temperature of 63°C to 68°C. The supersaturated $\alpha$-APM solution was fed through the heat exchanger 2 by the action of the peristaltic pump 3 at a rate of 25.8 L/min. A coolant at a temperature between 20 - 28 °C circulated through a cooling jacket of the heat exchanger 2. The $\alpha$-APM solution was cooled to a temperature of 56 - 58 °C by indirect heat-exchange with the coolant. The peristaltic pump 3 initiated the generation of $\alpha$-APM nuclei, forming a slurry, which is a mixture of the nuclei and supersaturated solution. In the transfer line 4 to the crystallizer 5, the crystals grew from the nuclei. The temperature of the slurry before entering the crystallizer 5 was 54 - 56 °C.

The crystallizer 5 is in a cylindrical shape with a diameter of about 2.3 m and a height of 3 m, and the internal volume is about 12 KL. The interior surface of the crystallizer 5 is made of stainless steel and surrounded with a cooling jacket. A coolant at a temperature of 4.5 - 5.0 °C continuously circulated in the cooling jacket. In this industrial scale crystallizer 5, a ribbon type impeller was installed. This impeller continuously rotated at about 11 rpm to mix the slurry contained in the crystallizer 5. Attached to the impeller was a scraper, the distal edge of which moves at a linear speed of about 79.5 m/min. The transfer line 4 was extended below the top surface of the slurry in the crystallizer 5 to prevent the slurry contacting the atmosphere. After 8-10 KL of the slurry was charged in the crystallizer 5, a continuous mode operation started by withdrawing the slurry at the same rate as it was being introduced thereto. The average residence time of slurry in the crystallizer 5 was about 390 min, and the temperature of the slurry under steady state conditions was in the range of 12 - 17 °C. The water content of the resulting wet crystals of $\alpha$-APM ranged from 40 to 60 wt.%.

Although this invention has been described in terms of a certain preferred embodiment, other embodiments will become apparent to those of ordinary skill in the art, in view of the disclosure herein. Accordingly, the present invention is not intended to be limited by the recitation of preferred embodiments, but is instead intended to be defined solely by reference to claims that follow.

**Claims**

1. A method of producing crystals of $\alpha$-L-aspartyl-L-phenylalanine methyl ester ("$\alpha$-APM"), comprising:

    forming a supersaturated solution of $\alpha$-APM; initiating nucleation of $\alpha$-APM crystal nuclei with use of a peristaltic pump, thereby forming a suspension of crystal nuclei in the supersaturated solution; and growing the $\alpha$-APM crystal nuclei in the supersaturated solution, without substantial formation of new crystal nuclei.

2. The method of claim 1, wherein the forming the supersaturated solution comprises:

    preparing a non-supersaturated $\alpha$-APM solution; and cooling the non-supersaturated solution to form the supersaturated solution.

3. The method of claim 2, wherein the concentration of the non-supersaturated $\alpha$-APM solution is in the range from about 1.5 wt.% to about 7.0 wt.%.

4. The method of claim 3, wherein the concentration of the non-supersaturated solution is from about 3.0 wt. % to about 5.5 wt. %.

5. The method of any one of claims 2 to 4, wherein the temperature of the non supersaturated $\alpha$-APM solution is in the range from about 35°C to about 85°C before cooling.

6. The method of claim 5, wherein the temperature of the non-supersaturated $\alpha$-APM solution is from about 55 °C to about 75 °C before cooling.

7. The method of any one of claims 2 to 6, wherein the cooling is conducted at less than a maximum allowable undercooling at a concentration.

8. The method of any one of claims 2 to 7, wherein the cooling is conducted by heat exchanging with a coolant.

9. The method of any one of claims 1 to 8, wherein the supersaturated solution is an aqueous solution.

10. The method of any one of claims 1 to 9, wherein the concentration of $\alpha$-APM in the supersaturated solution prior to initiating nucleation is greater than about 3.5 wt.%.

11. The method of any one of claims 1 to 10, wherein the initiating nucleation is conducted by mechanical forcing of the solution by the peristaltic pump.

12. The method of any one of claims 1 to 11, wherein the growing of the crystal nuclei is conducted without substantial formation of new crystal nuclei.

13. The method of claim 12, wherein the initiating nucleation comprises continuously feeding the solution to the peristaltic pump.

14. The method of any one of claims 1 to 10, wherein the initiating nucleation further comprises introducing seed crystals into the solution.

15. The method of any one of claims 1 to 14, wherein the initiating nucleation is conducted at a solution temperature ranging from about 28°C to about 80°C.

16. The method of claim 15, wherein the solution temperature is from about 48°C to about 66°C.

17. The method of any one of claims 1 to 16, wherein the growing the $\alpha$-APM crystal nuclei comprises a spontaneous growing stage after the initiation of nucleation and a forced growing stage after the spontaneous growing stage.

18. The method of claim 17, wherein the spontaneous growing stage proceeds at a supersaturated solution temperature ranging from about 45°C to about 60°C.

19. The method of claim 17 or claim 18, wherein the forced growing stage comprises cooling the suspension of crystal nuclei in the supersaturated solution.

20. The method of claim 19, wherein the cooling is conducted at a temperature ranging from about 5°C to about 60°C.

21. The method of claim 19 or claim 20, wherein the cooling is conducted in a batch mode operation.

22. The method of claim 19 or claim 20, wherein the cooling is conducted in a continuous mode operation.

23. The method of claim 19 or claim 20, wherein the cooling is conducted for a time of from about 50 to about 300 min.

24. The method of any one of claims 19 to 23, wherein the suspension of crystal nuclei in the supersaturated solution is stirred during the cooling.

25. The method of claim 24, wherein the stirring is conducted by an anchor type impeller or a ribbon type impeller.

26. The method of claim 25, wherein a scraper is attached to the impeller.

27. The method of any one of claims 24 to 26, wherein the stirring is conducted at from about 2 rpm to about 100 rpm.

28. The method of any one of claims 19 to 27, wherein the suspension of crystal nuclei in the supersaturated solution is not in contact with a gaseous atmosphere before the cooling of the forced growing stage.

29. The method of any one of claims 1 to 28, further comprising conducting a solid-liquid separation after growing the $\alpha$-APM crystal nuclei in the supersaturated solution to form $\alpha$-APM crystals.

30. The method of claim 29, further comprising drying the $\alpha$-APM crystals under elevated temperature.

31. The method of claim 1, wherein the method is performed in an industrial scale.

32. The method of any one of claims 1 to 31, wherein the growing of $\alpha$-APM crystal comprises cooling the suspension of crystal nuclei in the supersaturated solution in a container having a capacity greater than 1 KL.

33. A method according to any one of claims 1 to 32, wherein no further nucleation occurs in the suspension after the initiation of nucleation with the use of the peristaltic pump.

34. A method according to any one of claims 1 to 33, wherein the supersaturated solution subject to the nucleation is substantially free of crystals or crystal nuclei.

35. A method according to any one of claims 1 to 34, wherein crystal nuclei or grown crystals are fed to a crystallizer.

**36.** An apparatus for use in crystallization of α-APM dissolved in a solvent, the apparatus comprising:

a source of a supersaturated solution of α-APM;
a peristaltic pump in fluid communication with the supersaturated solution; and
a crystallizer in fluid communication with the pump.

**37.** The apparatus of claim 36, further comprising a transfer line in fluid communication with the source and the pump.

**38.** The apparatus of claim 37, wherein the transfer line is equipped with a heat exchanger.

**39.** The apparatus of any one of claims 36 to 38, wherein the crystallizer comprises a cooling container adapted to receive and cool a suspension of crystal nuclei from the pump.

**40.** The apparatus of claim 39, wherein the crystallizer comprises a heat exchanger.

**41.** The apparatus of any of claims 36 to 40, wherein the crystallizer further comprises a stirrer.

**42.** The apparatus of claim 41, wherein the stirrer comprises an anchor type impeller or a ribbon type impeller.

**43.** The apparatus of claim 41 or claim 42, wherein the stirrer comprises a a scraper.

**44.** The apparatus of any one of claims 36 to 43, further comprising a transfer line in fluid communication with the crystallizer and the pump.

**45.** The apparatus of any one of claims 36 to 44, wherein the crystallizer has a capacity greater than 1 KL.

**Patentansprüche**

**1.** Verfahren zur Herstellung von Kristallen aus α-L-Aspartyl-L-phenylalaninmethylester ("α-APM"), umfassend:

Bilden einer übersättigten Lösung aus α-APM;

Initiieren der Keimbildung aus α-APM-Kristallkeimen unter Verwendung einer peristaltischen Pumpe, wobei in der übersättigten Lösung eine Suspension aus Kristallkeimen gebildet wird; und

Züchten der α-APM-Kristallkeime in der übersättigten Lösung, ohne wesentliche Bildung neuer Kristallkeime.

**2.** Verfahren nach Anspruch 1, worin die Bildung der übersättigten Lösung Folgendes umfasst:

Herstellen einer nicht übersättigten α-APM-Lösung; und

Abkühlen der nicht übersättigten Lösung zur Bildung der übersättigten Lösung.

**3.** Verfahren nach Anspruch 2, worin die Konzentration der nicht übersättigten α-APM-Lösung im Bereich von ca. 1,5 Gew.-% bis ca. 7,0 Gew.-% liegt.

**4.** Verfahren nach Anspruch 3, worin die Konzentration der nicht übersättigten Lösung von ca. 3,0 Gew.-% bis ca. 5,5 Gew.-% beträgt.

**5.** Verfahren nach einem der Ansprüche 2 bis 4, worin die Temperatur der nicht übersättigten α-APM-Lösung vor dem Abkühlen im Bereich von ca. 35 °C bis ca. 85 °C liegt.

**6.** Verfahren nach Anspruch 5, worin die Temperatur der nicht übersättigten α-APM-Lösung vor dem Abkühlen von ca. 55 °C bis ca. 75 °C beträgt.

**7.** Verfahren nach einem der Ansprüche 2 bis 6, worin das Abkühlen bei weniger als einer maximal zulässigen Unterkühlung bei einer Konzentration durchgeführt wird.

**8.** Verfahren nach einem der Ansprüche 2 bis 7, worin das Abkühlen mittels Wärmeaustausch mit einem Kühlmittel durchgeführt wird.

**9.** Verfahren nach einem der Ansprüche 1 bis 8, worin die übersättigte Lösung eine wässrige Lösung darstellt.

**10.** Verfahren nach einem der Ansprüche 1 bis 9, worin die Konzentration von α-APM in der übersättigten Lösung vor der Initiierung der Keimbildung größer als ca. 3,5 Gew.-% ist.

**11.** Verfahren nach einem der Ansprüche 1 bis 10, worin die Initiierung der Keimbildung durch mechanisches Forcieren der Lösung mittels der peristaltischen Pumpe durchgeführt wird.

**12.** Verfahren nach einem der Ansprüche 1 bis 11, worin das Züchten der Kristallkeime ohne wesentliche Bildung neuer Kristallkeime durchgeführt wird.

**13.** Verfahren nach Anspruch 12, worin die Initiierung der Keimbildung ein kontinuierliches Zuspeisen der Lösung an die peristaltische Pumpe umfasst.

14. Verfahren nach einem der Ansprüche 1 bis 10, worin die Initiierung der Keimbildung weiter die Einführung von Impfkristallen in die Lösung umfasst.

15. Verfahren nach einem der Ansprüche 1 bis 14, worin die Initiierung der Keimbildung bei einer Temperatur der Lösung im Bereich von ca. 28 °C bis ca. 80 °C durchgeführt wird.

16. Verfahren nach Anspruch 15, worin die Temperatur der Lösung von ca. 48 °C bis ca. 66 °C beträgt.

17. Verfahren nach einem der Ansprüche 1 bis 16, worin das Züchten der α-APM-Kristallkeime ein spontanes Wachstumsstadium nach der Initiierung der Keimbildung und ein forciertes Wachstumsstadium nach dem spontanen Wachsstumsstadium umfasst.

18. Verfahren nach Anspruch 17, worin das spontane Wachstumsstadium bei einer Temperatur der übersättigten Lösung im Bereich von ca. 45 °C bis ca. 60 °C abläuft.

19. Verfahren nach Anspruch 17 oder 18, worin das forcierte Wachstumsstadium das Abkühlen der Suspension aus Kristallkeimen in der übersättigten Lösung umfasst.

20. Verfahren nach Anspruch 19, worin das Abkühlen bei einer Temperatur im Bereich von ca. 5 °C bis ca. 60 °C durchgeführt wird.

21. Verfahren nach Anspruch 19 oder 20, worin das Abkühlen in einer diskontinuierlichen Betriebsweise durchgeführt wird.

22. Verfahren nach Anspruch 19 oder 20, worin das Abkühlen in einer kontinuierlichen Betriebsweise durchgeführt wird.

23. Verfahren nach Anspruch 19 oder 20, worin das Abkühlen für eine Zeitdauer von 50 bis ca. 300 min durchgeführt wird.

24. Verfahren nach einem der Ansprüche 19 bis 23, worin die Suspension der Kristallkeime in der übersättigten Lösung während des Abkühlens gerührt wird.

25. Verfahren nach Anspruch 24, worin das Rühren mittels eines Anker-Schnellrührers oder eines Band-Schnellrührers durchgeführt wird.

26. Verfahren nach Anspruch 25, worin an dem Schnellrührer ein Schaber angebracht ist.

27. Verfahren nach einem der Ansprüche 24 bis 26, worin das Rühren bei von ca. 2 U/min bis ca. 100 U/min durchgeführt wird.

28. Verfahren nach einem der Ansprüche 19 bis 27, worin sich die Suspension aus Kristallkeimen in der übersättigten Lösung vor dem Abkühlen des forcierten Wachstumsstadiums nicht mit einer Gasatmosphäre in Kontakt befindet.

29. Verfahren nach einem der Ansprüche 1 bis 28, das weiter das Durchführen einer Fest-Flüssig-Trennung nach dem Züchten der α-APM-Kristallkeime in der übersättigten Lösung zur Bildung von α-APM-Kristallen umfasst.

30. Verfahren nach Anspruch 29, das weiter das Trocknen der α-AMP-Kristalle unter erhöhter Temperatur umfasst.

31. Verfahren nach Anspruch 1, worin das Verfahren im industriellen Maßstab durchgeführt wird.

32. Verfahren nach einem der Ansprüche 1 bis 31, worin das Züchten von α-AMP-Kristallen das Abkühlen der Suspension aus Kristallkeimen in der übersättigten Lösung in einem Behälter mit einer Kapazität von größer als 1 kl umfasst.

33. Verfahren nach einem der Ansprüche 1 bis 32, worin nach der Initiierung der Keimbildung unter Verwendung der peristaltischen Pumpe keine weitere Keimbildung in der Suspension auftritt.

34. Verfahren nach einem der Ansprüche 1 bis 33, worin die übersättigte Lösung abhängig von der Keimbildung im Wesentlichen frei von Kristallen oder Kristallkeimen ist.

35. Verfahren nach einem der Ansprüche 1 bis 34, worin die Kristallkeime oder gezüchteten Kristalle an einen Kristallisator gespeist werden.

36. Apparat zur Verwendung bei der Kristallisation von in einem Lösungsmittel aufgelöstem α-APM, wobei der Apparat Folgendes umfasst:

eine Quelle einer übersättigten Lösung aus α-APM;

eine peristaltische Pumpe in flüssiger Kommunikation mit der übersättigten Lösung; und

einen Kristallisator in flüssiger Kommunikation mit der Pumpe.

37. Apparat nach Anspruch 36, der weiter eine Transferleitung in flüssiger Kommunikation mit der Quelle und der Pumpe umfasst.

**38.** Apparat nach Anspruch 37, worin die Transferleitung mit einem Wärmeaustauscher ausgerüstet ist.

**39.** Apparat nach einem der Ansprüche 36 bis 38, worin der Kristallisator einen Kühlungsbehälter umfasst, der zur Aufnahme und zum Abkühlen einer Suspension aus Kristallkeimen aus der Pumpe angepasst ist.

**40.** Apparat nach Anspruch 39, worin der Kristallisator einen Wärmeaustauscher umfasst.

**41.** Apparat nach einem der Ansprüche 36 bis 40, worin der Kristallisator weiter einen Rührer umfasst.

**42.** Apparat nach Anspruch 41, worin der Rührer einen Anker-Schnellrührer oder einen Band-Schnellrührer umfasst.

**43.** Apparat nach Anspruch 41 oder 42, worin der Rührer einen Schaber umfasst.

**44.** Apparat nach einem der Ansprüche 36 bis 43, der weiter eine Transferleitung in flüssiger Kommunikation mit dem Kristallisator und der Pumpe umfasst.

**45.** Apparat nach einem der Ansprüche 36 bis 44, worin der Kristallisator eine Kapazität von größer als 1 kl aufweist.

## Revendications

**1.** Procédé de production de cristaux de $\alpha$-L-aspartyl-L-phénylalanine méthyl ester ("$\alpha$-APM") comprenant :

la formation d'une solution supersaturée de $\alpha$-APM ;
le lancement d'une nucléation de noyaux de cristaux de $\alpha$-APM au moyen d'une pompe péristaltique, formant ainsi une suspension de noyaux de cristaux dans la solution supersaturée ; et
la croissance des noyaux de cristaux de $\alpha$-APM dans la solution supersaturée, sans formation substantielle de nouveaux noyaux de cristaux.

**2.** Procédé selon la revendication 1, dans lequel la formation de la solution supersaturée comprend :

la préparation d'une solution de $\alpha$-APM non supersaturée ; et
le refroidissement de la solution non supersaturée afin de former la solution supersaturée.

**3.** Procédé selon la revendication 2, dans lequel la concentration de la solution de $\alpha$-APM non supersaturée est comprise dans la gamme d'environ 1,5 % en poids à environ 7,0 % en poids.

**4.** Procédé selon la revendication 3, dans lequel la concentration de la solution non supersaturée est comprise dans la gamme d'environ 3,0 % en poids à environ 5,5 % en poids.

**5.** Procédé selon l'une quelconque des revendications 2 à 4, dans lequel la température de la solution de $\alpha$-APM non supersaturée est comprise dans la gamme d'environ 35 °C à environ 85 °C avant refroidissement.

**6.** Procédé selon la revendication 5, dans lequel la température de la solution $\alpha$-APM non supersaturée est comprise dans la gamme d'environ 55 °C à environ 75 °C avant refroidissement.

**7.** Procédé selon l'une quelconque des revendications 2 à 6, dans lequel le refroidissement est effectué à une température inférieure à un sous-refroidissement admissible maximum à une concentration.

**8.** Procédé selon l'une quelconque des revendications 2 à 7, dans lequel le refroidissement est effectué par échange thermique avec un réfrigérant.

**9.** Procédé selon l'une quelconque des revendications 1 à 8, dans lequel la solution supersaturée est une solution aqueuse.

**10.** Procédé selon l'une quelconque des revendications 1 à 9, dans lequel la concentration de $\alpha$-APM dans la solution supersaturée avant le lancement de la nucléation est supérieure à environ 3,5 % en poids.

**11.** Procédé selon l'une quelconque des revendications 1 à 10, dans lequel le lancement de la nucléation est effectué par un forçage mécanique de la solution par la pompe péristaltique.

**12.** Procédé selon l'une quelconque des revendications 1 à 11, dans lequel la croissance des noyaux de cristaux est effectuée sans formation substantielle de nouveaux noyaux de cristaux.

**13.** Procédé selon la revendication 12, dans lequel le lancement de la nucléation comprend l'alimentation continue de la solution à la pompe péristaltique.

**14.** Procédé selon l'une quelconque des revendications 1 à 10, dans lequel le lancement de la nucléation comprend en outre l'introduction de germes cristallins dans la solution.

**15.** Procédé selon l'une quelconque des revendications 1 à 14, dans lequel le lancement de la nucléation est effectué à une température de solution allant d'environ 28 °C à environ 80 °C.

**16.** Procédé selon la revendication 15, dans lequel la température de solution est comprise dans la gamme d'environ 48 °C à environ 66 °C.

**17.** Procédé selon l'une quelconque des revendications 1 à 16, dans lequel la croissance des noyaux de cristaux de α-APM comprend un stade de croissance spontanée après le lancement de la nucléation et un stade de croissance forcée après le stade de croissance spontanée.

**18.** Procédé selon la revendication 17, dans lequel le stade de croissance spontanée se déroule à une température de solution supersaturée allant d'environ 45 °C à environ 60 °C.

**19.** Procédé selon la revendication 17 ou la revendication 18, dans lequel le stade de croissance forcée comprend le refroidissement de la suspension de noyaux de cristaux dans la solution supersaturée.

**20.** Procédé selon la revendication 19, dans lequel le refroidissement est effectué à une température allant d'environ 5 °C à environ 60 °C.

**21.** , Procédé selon la revendication 19 ou la revendication 20, dans lequel le refroidissement est effectué en un mode par lots.

**22.** Procédé selon la revendication 19 ou la revendication 20, dans lequel le refroidissement est effectué en un mode continu.

**23.** Procédé selon la revendication 19 ou la revendication 20, dans lequel le refroidissement est effectué pendant une durée allant d'environ 50 à environ 300 minutes.

**24.** Procédé selon l'une quelconque des revendications 19 à 23, dans lequel la suspension des noyaux de cristaux dans la solution supersaturée est agitée durant le refroidissement.

**25.** Procédé selon la revendication 24, dans lequel l'agitation est effectuée par un mélangeur forme ancre ou un mélangeur à vis hélicoïdale.

**26.** Procédé selon la revendication 25, dans lequel une racle est fixée au mélangeur.

**27.** Procédé selon l'une quelconque des revendications 24 à 26, dans lequel l'agitation est effectuée à d'environ 2 tours/minute à 100 tours/minute.

**28.** Procédé selon l'une quelconque des revendications 19 à 27, dans lequel la suspension de noyaux de cristaux dans la solution supersaturée n'est pas en contact avec une atmosphère gazeuse avant le refroidissement du stade de croissance forcée.

**29.** Procédé selon l'une quelconque des revendications 1 à 28, comprenant en outre la réalisation d'une séparation solides-liquide après la croissance des noyaux de cristaux de α-APM dans la solution supersaturée afin de former des cristaux de α-APM.

**30.** Procédé selon la revendication 29, comprenant en outre le séchage des cristaux de α-APM à une température élevée.

**31.** Procédé selon la revendication 1, dans lequel le procédé est effectué à échelle industrielle.

**32.** Procédé selon l'une quelconque des revendications 1 à 31, dans lequel la croissance du cristal de α-APM comprend le refroidissement de la suspension de noyaux de cristaux dans la solution supersaturée dans un récipient ayant une capacité supérieure à 1 KL.

**33.** Procédé selon l'une quelconque des revendications 1 à 32, dans lequel aucune autre nucléation n'intervient dans la suspension après le lancement de la nucléation au moyen de la pompe péristaltique.

**34.** Procédé selon l'une quelconque des revendications 1 à 33, dans lequel la solution supersaturée soumise à la nucléation est substantiellement dépourvue de cristaux ou de noyaux de cristaux.

**35.** Procédé selon l'une quelconque des revendications 1 à 34, dans lequel les noyaux de cristaux ou cristaux crûs sont passés à un cristalliseur.

**36.** Appareil destiné à être utilisé dans la cristallisation de α-APM dissout dans un solvant, l'appareil comprenant :

une source de solution supersaturée de α-APM ;
une pompe péristaltique en communication fluide avec la solution supersaturée ; et
un cristalliseur en communication fluide avec la pompe.

**37.** Appareil selon la revendication 36, comprenant en outre une conduite de transfert en communication fluide avec la source et la pompe.

**38.** Appareil selon la revendication 37, dans lequel la conduite de transfert est équipée d'un échangeur thermique.

**39.** Appareil selon l'une quelconque des revendications 36 à 38, dans lequel le cristalliseur comprend un récipient de refroidissement adapté pour recevoir et refroidir une suspension de noyaux de cristaux provenant de la pompe.

**40.** Appareil selon la revendication 39, dans lequel le cristalliseur comprend un échangeur thermique.

**41.** Appareil selon l'une quelconque des revendications 36 à 40, dans lequel le cristalliseur comprend en outre un agitateur.

**42.** Appareil selon la revendication 41, dans lequel l'agitateur comprend un mélangeur forme ancre ou un mélangeur à vis hélicoïdale.

**43.** Appareil selon la revendication 41 ou la revendication 42, dans lequel l'agitateur comprend une racle.

**44.** Appareil selon l'une quelconque des revendications 36 à 43, comprenant en outre une conduite de transfert en communication fluide avec le cristalliseur et la pompe.

**45.** Appareil selon l'une quelconque des revendications 36 à 44, dans lequel le cristalliseur a une capacité supérieure à 1 KL.

FIG. 1

FIG.2

FIG.3

EP 1 077 216 B1

FIG.4